(19) Europäisches Patentamt
European Patent Office
Office européen des brevets

(11) Veröffentlichungsnummer: **0 005 780**
**A1**

# (12) EUROPÄISCHE PATENTANMELDUNG

(21) Anmeldenummer: 79101538.1

(22) Anmeldetag: 21.05.79

(51) Int. Cl.²: C 07 D 249/08
A 01 N 9/22

(30) Priorität: 03.06.78 DE 2824394

(43) Veröffentlichungstag der Anmeldung:
12.12.79 Patentblatt 79/25

(84) Benannte Vertragsstaaten:
BE CH DE FR GB IT NL

(71) Anmelder: BAYER Aktiengesellschaft
Zentralbereich Patente,Marken und Lizenzen Bayerwerk
D-5090 Leverkusen 1(DE)

(72) Erfinder: Stetter, Jörg, Dr.
Pahlkestrasse 3
D-5600 Wuppertal 1(DE)

(72) Erfinder: Homeyer, Bernhard, Dr.
Obere Strasse 28
D-5090 Leverkusen 3(DE)

(72) Erfinder: Hammann, Ingeborg, Dr.
Belfortstrasse 9
D-5000 Köln 1(DE)

## (54) N,N'-unsymmetrisch substituierte Thio-bis-amine, Verfahren zu ihrer Herstellung und ihre Verwendung als Insektizide, Akarizide und Nematozide.

(57) Die vorliegende Erfindung betrifft N,N'-unsymmetrisch substituierte Thio-bis-amine der allgemeinen Formel I

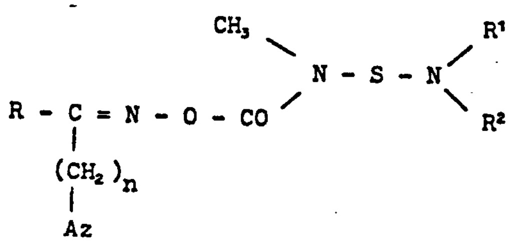

(I)

in welcher $A_2$, R, $R^1$, $R^2$ in die in der Beschreibung angegebene Bedeutung besitzen.

Man erhält sie, wenn man

a) Oxime der Formel

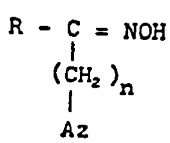

(II)

mit sulfenylierten Carbamoylhalogeniden der Formel

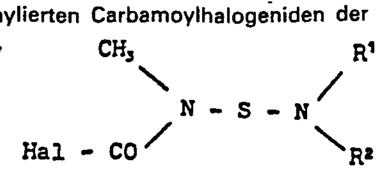

(III)

umsetzt, oder

b) gegebenenfalls die nach dem Verfahren (a) erhältlichen sulfenylierten Oximcarbamate der Formel

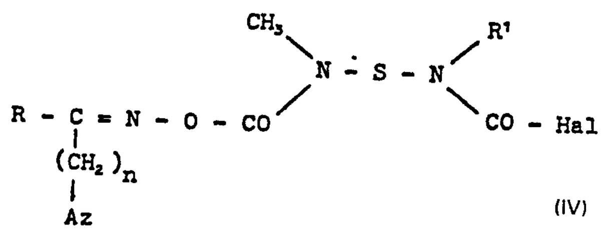

(IV)

mit Verbindungen der Formel

$H - R^8$

umsetzt.

Die erfindungsgemäßen Verbindungen zeigen insektizide, akarizide und nematozide Wirkung.

EP 0 005 780 A1

BAYER AKTIENGESELLSCHAFT
Zentralbereich
Patente, Marken und Lizenzen

5090 Leverkusen, Bayerwerk
Rt/kü
Typ Ib

N,N'-unsymmetrisch substituierte Thio-bis-amine, Verfahren zu ihrer Herstellung und ihre Verwendung als Insektizide, Akarizide und Nematozide

Die vorliegende Erfindung betrifft neue N,N'-unsymmetrisch substituierte Thio-bis-amine, mehrere Verfahren zu ihrer Herstellung und ihre Verwendung als Insektizide, Akarizide und Nematozide.

Es ist bereits bekannt geworden, daß N-sulfenylierte Oximcarbamate gute insektizide, akarizide und nematozide Eigenschaften aufweisen (vergleiche DE-OS 2 635 883 [LeA 17 354]). Deren Wirkung ist jedoch, insbesondere bei niedrigen Aufwandmengen, nicht immer ganz befriedigend.

Es wurden neue N,N'-unsymmetrisch substituierte Thio-bis-amine der allgemeinen Formel I gefunden,

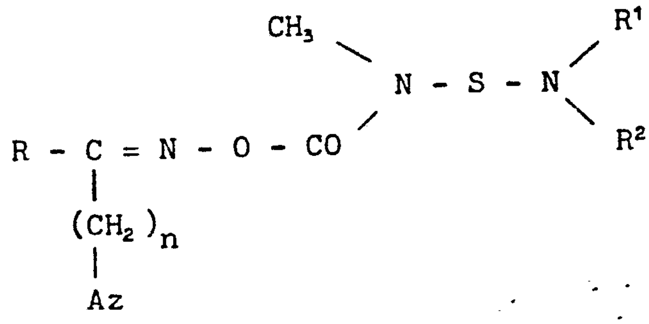

(I)

in welcher

Az für einen gegebenenfalls substituierten Azolylrest steht,

R für Alkyl steht,

$R^1$ für Alkyl steht,

$R^2$ für Alkyl, Halogencarbonyl oder die Gruppierungen $-CO-O-R^3$ und $-CO-NR^4R^5$ steht sowie

$R^1$ und $R^2$ gemeinsam mit dem angrenzenden N-Atom für einen Ring stehen, der gegebenenfalls ein weiteres Heteroatom enthält, wobei $R^1$ für Methyl stehen muß, wenn $R^2$ für Halogencarbonyl steht,

$R^3$ für Alkyl, Alkenyl, Alkinyl, gegebenenfalls substituiertes Aryl oder die Gruppierung $-N=CR^6R^7$ steht,

$R^4$ und $R^5$ gleich oder verschieden sind und für Alkyl stehen oder gemeinsam mit dem angrenzenden N-Atom für einen Ring stehen, der gegebenenfalls ein weiteres Heteroatom enthält,

$R^6$ und $R^7$ gleich oder verschieden sind und für Wasserstoff, Alkyl, Alkoxy, Alkylthio, Alkoxyalkyl, Alkylthioalkyl, Dialkylaminocarbonyl und Alkoxycarbonyl oder gemeinsam mit dem angrenzenden C-Atom für einen Dithian-Ring stehen, und

n für 0 oder 1 steht,

sowie deren physiologisch verträgliche Säureadditions-Salze und Metallsalz-Komplexe. Diese Verbindungen weisen starke insektizide, akarizide und nematozide Eigenschaften auf.

Die Verbindungen der Formel (I) können in der syn- oder anti-Form vorliegen; vorwiegend fallen sie als Gemische beider Formen an.

Man erhält die N,N'-unsymmetrisch substituierten Thio-bis-amine der Formel (I), wenn man

a) Oxime der Formel

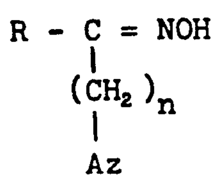

(II)

in welcher

Az, R und n die oben angegebene Bedeutung haben,

mit sulfenylierten Carbamoylhalogeniden der Formel

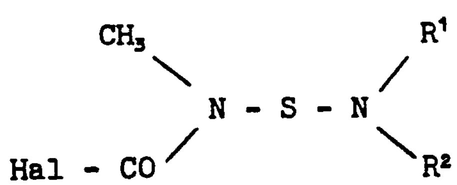

(III)

in welcher

$R^1$ und $R^2$ die oben angegebene Bedeutung haben, wobei aber $R^1$ nur für Methyl steht, wenn $R^2$ für Halogencarbonyl steht, und

Hal für Fluor oder Chlor steht,

in Gegenwart eines Verdünnungsmittels und eines Säurebindemittels umsetzt, oder

b) gegebenenfalls die nach dem Verfahren (a) erhältlichen sulfenylierten Oximcarbamate der Formel

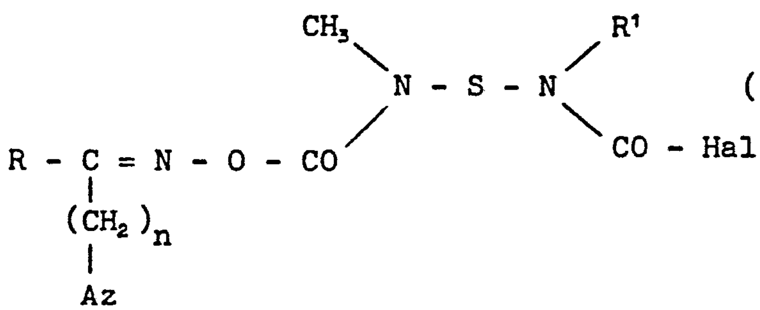

(IV)

in welcher

Az, R, $R^1$,
Hal und n - die oben angegebene Bedeutung haben,

mit Verbindungen der Formel

$H - R^8$ (V)

in welcher

$R^8$ für die Gruppierungen $-O-R^3$ und $-NR^4R^5$ steht und $R^3$, $R^4$ und $R^5$ die oben angegebene Bedeutung haben,

in Gegenwart eines Verdünnungsmittels und eines Säurebindemittels umsetzt.

Weiterhin können die erfindungsgemäß erhältlichen N,N'-unsymmetrisch substituierten Thio-bis-amine der Formel(I) durch Umsetzung mit Säuren in die Salze überführt werden, bzw. durch Reaktion mit Metallsalzen die entsprechenden Metallsalz-Komplexe erhalten werden.

Überraschenderweise zeigen die erfindungsgemäßen N,N'-unsymmetrisch subsituierten Thio-bis-amine eine höhere insektizide, akarizide und nematozide Wirkung als die bekannten N-sulfenylierten Oximcarbamate, welche chemisch und wirkungsmäßig naheliegende Verbindungen sind.

Die erfindungsgemäßen N,N'-unsymmetrisch substituierten Thio-bis-amine sind durch die Formel (I) allgemein definiert. In dieser Formel steht Az vorzugsweise für die gegebenenfalls substituierten Azolyl-Reste Pyrazol-1-yl, Imidazol-1-yl, 1,2,4-Triazol-1-yl, 1,2,3-Triazol-1-yl, 1,3,4-Triazol-1-yl, Indazol-1-yl, Benzimidazol-1-yl und Benztriazol-1-yl, wobei als Substituenten vorzugsweise infrage kommen: Halogen, insbesondere Fluor, Chlor und Brom, Alkyl mit 1 bis 4 Kohlenstoffatomen, Halogenalkyl mit bis zu 2 Kohlenstoff- und bis zu 5 Halogenatomen, wie insbesondere Fluor- und Chloratomen, beispielsweise sei Trifluormethyl genannt, Alkoxy und Alkylthio mit jeweils bis zu 4 Kohlenstoffatomen und die Nitrogruppe. R steht vorzugsweise für geradkettiges oder verzweigtes Alkyl mit 1 bis 4 Kohlenstoffatomen. $R^1$ steht vorzugsweise für geradkettiges oder verzweigtes Alkyl mit 1 bis 4 Kohlenstoffatomen. $R^2$ steht vorzugsweise für geradkettiges oder verzweigtes Alkyl mit 1 bis 4 Kohlenstoffatomen, Halogencarbonyl mit Fluor und Chlor als Halogenatome, sowie die Gruppierungen $-CO-O-R^3$ und $-CO-NR^4R^5$. $R^1$ und $R^2$ stehen außerdem zusammen mit dem angrenzenden N-Atom noch vorzugsweise für einen 5- bis 7-gliedrigen Ring, der gegebenenfalls ein weiteres Heteroatom enthalten kann, wie insbesondere Sauerstoff oder Stickstoff. $R^3$ steht vorzugsweise für Alkyl mit 1 bis 4 Kohlenstoffatomen, Alkenyl und Alkinyl mit jeweils 3 bis 4 Kohlenstoffatomen, sowie gegebenenfalls einfach oder mehrfach, gleich oder verschieden substituiertes Aryl mit 6 bis 10 Kohlenstoffatomen, wobei als Substituenten vorzugsweise infrage kommen: Halogen, geradkettiges oder verzweigtes Alkyl mit 1 bis 5 Kohlenstoffatomen, Alkoxy und Alkylthio mit jeweils 1 bis 4

Kohlenstoffatomen, Dialkylamino mit 1 bis 4 Kohlenstoffatomen in jedem Alkylteil, Dialkenylamino mit 2 bis 4 Kohlenstoffatomen in jedem Alkenylteil, Alkylthioalkyl mit 1 bis 2 Kohlenstoffatomen in jedem Alkylteil sowie gegebenenfalls ein- oder mehrfach durch Alkyl mit 1 bis 2 Kohlenstoffatomen substituiertes Dioxolanyl. $R^3$ steht außerdem vorzugsweise für gegebenenfalls ein- oder mehrfach durch Alkyl mit 1 bis 2 Kohlenstoffatomen substituiertes Benzofuranyl und Benzodioxolyl. $R^3$ steht weiterhin vorzugsweise für die Gruppierung $-N=CR^6R^7$.
$R^4$ und $R^5$ sind gleich oder verschieden und stehen vorzugsweise für Alkyl mit 1 oder 2 Kohlenstoffatomen sowie gemeinsam mit dem N-Atom für einen 5- bis 7-gliedrigen Ring, der gegebenenfalls ein weiteres Heteroatom enthalten kann.
$R^6$ und $R^7$ sind gleich oder verschieden und stehen vorzugsweise für Wasserstoff, geradkettiges oder verzweigtes Alkyl mit 1 bis 4 Kohlenstoffatomen; Alkylthio, Alkoxy, Alkylthioalkyl und Alkoxyalkyl mit jeweils 1 bis 4 Kohlenstoffatomen in jedem Alkylteil, Dialkylaminocarbonyl mit 1 bis 2 Kohlenstoffatomen in jedem Alkylteil sowie Alkoxycarbonyl mit 1 bis 4 Kohlenstoffatomen im Alkylteil. $R^6$ und $R^7$ stehen außerdem zusammen mit dem C-Atom noch vorzugsweise für einen Dithian-Ring.
Der Index n steht vorzugsweise für 0 und 1.

Ganz besonders bevorzugt sind diejenigen N,N'-unsymmetrisch substituierten Thio-bis-amine der Formel (I), in denen Az für gegebenenfalls durch Chlor, Methyl, Aethyl, Nitro oder Methylmercapto substituiertes Pyrazol-1-yl, Imidazol-1-yl, 1,2,4-Triazol-1-yl, 1,2,3-Triazol-1-yl, 1,3,4-Triazol-1-yl steht; R für tert.-Butyl oder iso-Propyl steht; $R^1$ für Methyl steht; $R^2$ für Methyl, Fluorcarbonyl sowie die Gruppierungen $-CO-O-R^3$ und $-CO-NR^4R^5$ steht; $R^1$ und $R^2$ zusammen mit dem N-Atom für Piperidino oder Morpholino stehen; $R^3$ für Methyl, Aethyl, Allyl, Propargyl, 2,2-Dimethylbenzofuran-7-yl, 2,2-Dimethyl-1,3-benzodioxol-4-yl, Naphth-1-yl und gegebenenfalls einfach oder mehrfach gleich oder verschieden substituiertes Phenyl steht, wobei als Substituenten besonders bevorzugt infrage kommen: Chlor, Methyl, Aethyl, i-Propyl, i-Butyl, sek.-Butyl, tert.-Butyl, Pent-2-yl, Pent-3-yl, Methoxy, Methylmercapto, i-Propoxy, i-Propylmercapto, Methoxymethyl, Aethoxymethyl, Methylthiomethyl, Aethylthiomethyl, Dimethylamino, Diallylamino und 4,5-Dimethyl-1,3-dioxolan-2-yl; $\underline{R^3}$ weiterhin für die Gruppierung $-N=CR^6R^7$ steht; $\underline{R^4}$ und $\underline{R^5}$ für Methyl oder zusammen mit dem N-Atom für Piperidino und Morpholino stehen; $\underline{R^6}$ und $\underline{R^7}$ gleich oder verschieden sind und für Wasserstoff, Methyl, i-Propyl, tert.-Butyl, Methoxy, Methylmercapto, Methoxymethyl, Methylthiomethyl, Dimethylcarbamoyl und Methoxycarbonyl stehen, oder gemeinsam mit dem C-Atom für einen 1,3-Dithian-Ring stehen und der Index $\underline{n}$ für 0 und 1 steht.

Im Einzelnen seien außer den Herstellungsbeispielen und den Beispielen der Tabelle 1 folgende Verbindungen genannt:

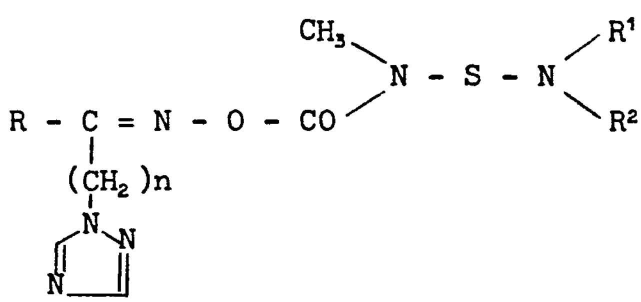

| n | R | $R^1$ | $R^2$ |
|---|---|---|---|
| 1 | $C(CH_3)_3$ | 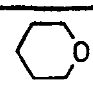 | |
| 1 | $C(CH_3)_3$ | $CH_3$ | $-CO-O-N=C-SCH_3$<br>$CH_3$ |
| 1 | $C(CH_3)_3$ | $CH_3$ | $-CO-F$ |
| 1 | $C(CH_3)_3$ | $CH_3$ | $-CO-O-N=C-CO-N(CH_3)_2$<br>$SCH_3$ |
| 1 | $C(CH_3)_3$ | $CH_3$ | $-CO-O-C_2H_5$ |
| 1 | $C(CH_3)_3$ | $CH_3$ | $-CO-O-CH_3$ |
| 0 | $C(CH_3)_3$ | $CH_3$ | $-CO-O-N=C-C(CH_3)_2-SCH_3$<br>H |

| n | R | $R^1$ | $R^2$ |
|---|---|---|---|
| 0 | $C(CH_3)_3$ | $CH_3$ | $-CO-O-N=C-C(CH_3)_3$<br>$SCH_3$ |
| 0 | $C(CH_3)_3$ | $CH_3$ | $-CO-O-N=$ 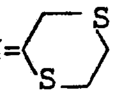 |
| 0 | $C(CH_3)_3$ | $CH_3$ | $-CO-O-N=C-C(CH_3)_3$<br>$CH_2-S-CH_3$ |
| 0 | $C(CH_3)_3$ | $CH_3$ | $-CO-O-N=C-CH_3$<br>$CH_3$ |
| 0 | $C(CH_3)_3$ | $CH_3$ | $-CO-O-N=C-C(CH_3)_3$<br>$OCH_3$ |
| 0 | $C(CH_3)_3$ | $CH_3$ | $-CO-O-$ 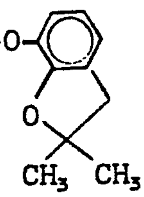 |
| 0 | $C(CH_3)_3$ | $CH_3$ | $-CO-O-$ 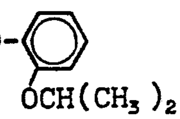 |
| 0 | $C(CH_3)_3$ | $CH_3$ | $-CO-O-$ 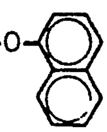 |
| 0 | $-CH(CH_3)_2$ | $CH_3$ | $-CO-O-N=C-CH_3$<br>$SCH_3$ |
| 0 | $-CH(CH_3)_2$ | $CH_3$ | $-CO-O-N=C-SCH_3$<br>$CO-N(CH_3)_2$ |
| 0 | $C(CH_3)_3$ | $CH_3$ | $CH_3$ |
| 0 | $C(CH_3)_3$ | $CH_3$ | $-CO-O-CH_2-C\equiv CH$ |
| 0 | $-CH(CH_3)_2$ | 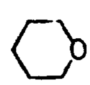 | |
| 0 | $-CH(CH_3)_2$ | $CH_3$ | $-CO-O-CH_3$ |
| 0 | $-CH(CH_3)_2$ | $CH_3$ | $-CO-O-CH_2-C\equiv CH$ |

| n | R | R¹ | R² |
| --- | --- | --- | --- |
| 1 | $C(CH_3)_3$ | $CH_3$ | $-CO-O-N=C-C(CH_3)_3$<br>$SCH_3$ |
| 1 | $C(CH_3)_3$ | $CH_3$ | $-CO-O-N=$ 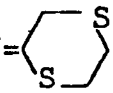 |
| 1 | $C(CH_3)_3$ | $CH_3$ | $-CO-O-N=C-C(CH_3)_3$<br>$CH_2-S-CH_3$ |
| 1 | $C(CH_3)_3$ | $CH_3$ | $-CO-O-N=C-CH_3$<br>$CH_3$ |
| 1 | $C(CH_3)_3$ | $CH_3$ | $-CO-O-N=C-C(CH_3)_3$<br>$OCH_3$ |
| 1 | $C(CH_3)_3$ | $CH_3$ | $-CO-O-$ 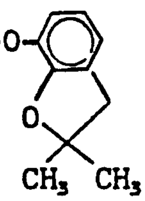 |
| 1 | $C(CH_3)_3$ | $CH_3$ | $-CO-O-$ 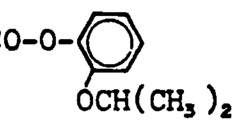 |
| 1 | $C(CH_3)_3$ | $CH_3$ | $-CO-O-$ 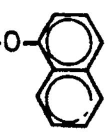 |
| 1 | $-CH(CH_3)_2$ | $CH_3$ | $-CO-O-N=C-CH_3$<br>$SCH_3$ |
| 1 | $-CH(CH_3)_2$ | $CH_3$ | $-CO-O-N=C-SCH_3$<br>$CO-N(CH_3)_2$ |
| 1 | $C(CH_3)_3$ | $CH_3$ | $CH_3$ |
| 1 | $C(CH_3)_3$ | $CH_3$ | $-CO-O-CH_2-C\equiv CH$ |
| 1 | $-CH(CH_3)_2$ | 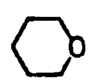 | |
| 1 | $-CH(CH_3)_2$ | $CH_3$ | $-CO-O-CH_3$ |
| 1 | $-CH(CH_3)_2$ | $CH_3$ | $-CO-O-CH_2-C\equiv CH$ |
| 1 | $C(CH_3)_3$ | $CH_3$ | $-CO-O-N=C-C(CH_2)_2-SCH_3$<br>$H$ |

Verwendet man 2,2-Dimethyl-1-oximino-1-(1,2,4-triazol-1-yl)-propan und N-Methyl-N-morpholin-4-yl-sulfenyl-carbamoylfluorid als Ausgangsstoffe, so kann der Reaktionsablauf durch das folgende Formelschema wiedergegeben werden (Verfahren a):

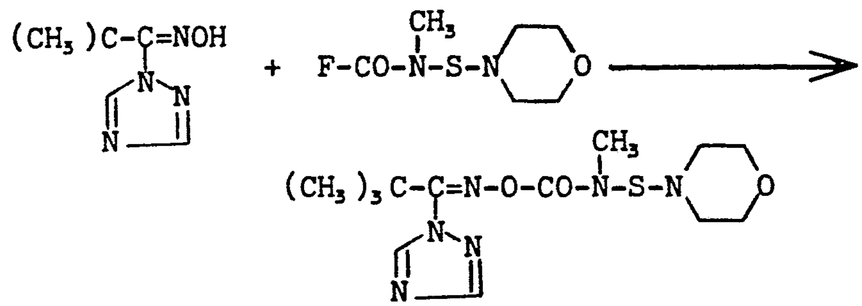

Verwendet man 3,3-Dimethyl-2-oximino-1-(1,2,4-triazol-1-yl)-butan und 1-Methylthioacetaldehyd-O-[N-methyl-N-(N'-methyl-N'-fluorformyl-aminosulfenyl)-carbamoyl]-oxim als Ausgangsstoffe, so kann der Reaktionsablauf durch das folgende Formelschema wiedergegeben werden (Verfahren a):

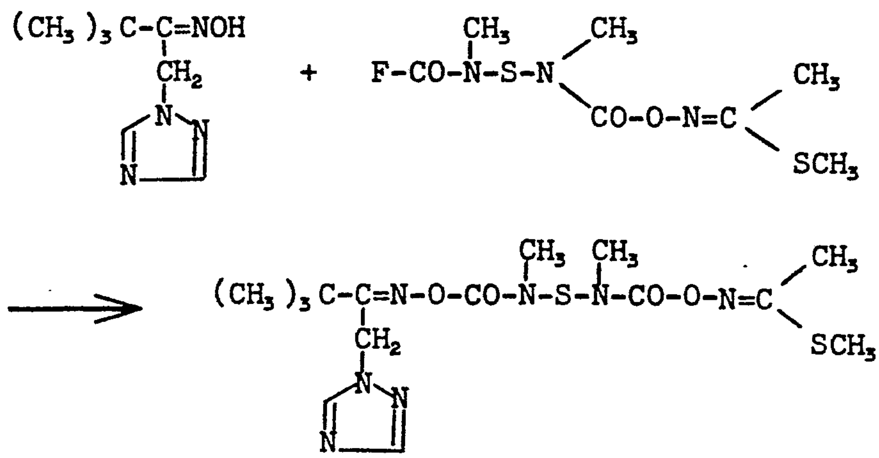

Verwendet man 2,2-Dimethyl-1-oximino-1-(1,2,4-triazol-1-yl)-propan und N,N'-Bis-(fluorcarbonyl)-thio-bis-methylamin als Ausgangsstoffe, so kann der Reaktionsablauf durch das folgende Formelschema wiedergegeben werden (Verfahren a):

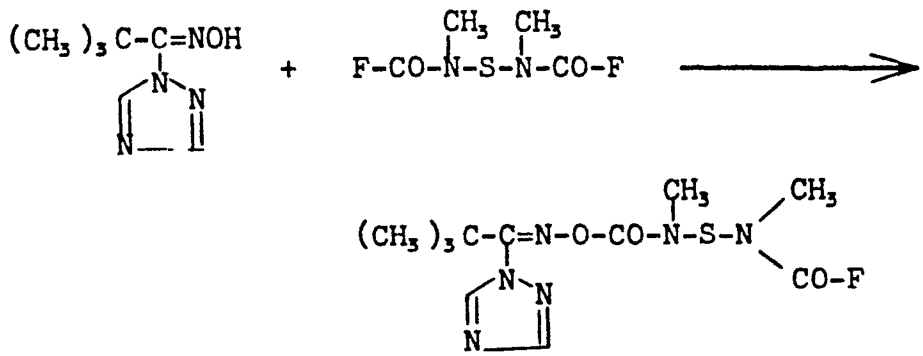

Verwendet man 2,2-Dimethyl-1-(1,2,4-triazol-1-yl)-propanaldehyd-O-[N-methyl-N-(N'-methyl-N'-fluorformyl-aminosulfenyl)-carbamoyl]-oxim und Methanol als Ausgangsstoffe, so kann der Reaktionsablauf durch das folgende Formelschema wiedergegeben werden (Verfahren b):

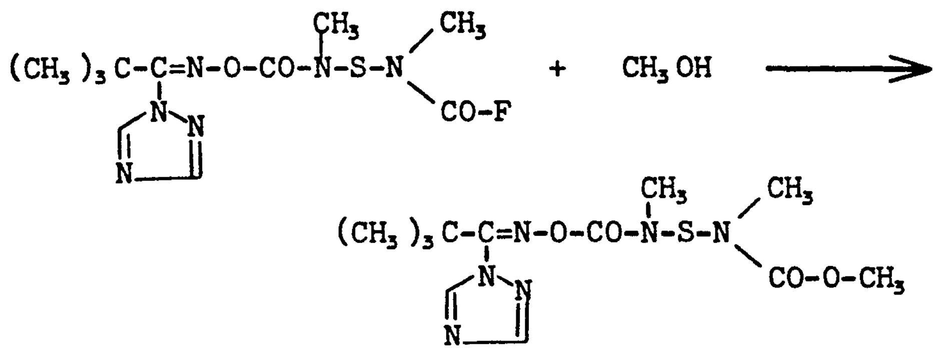

Die für Verfahren (a) als Ausgangsstoffe zu verwendenden Oxime sind durch die Formel (II) allgemein definiert. In dieser Formel stehen Az, R und n vorzugsweise für die Reste, die bei den Verbindungen der Formel (I) bereits vorzugsweise genannt wurden.

Die Oxime der Formel (II) sind noch nicht bekannt, können jedoch auf bekannte Art und Weise erhalten werden (vergleiche hierzu DE-OS 2 635 883).
Die Oxime der Formel (II), in denen n für 0 steht, können hergestellt werden, indem man Hydroxamsäurehalogenide der Formel

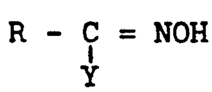 (VI)

in welcher

R die oben angegebene Bedeutung hat und

Y für Halogen, insbesondere Chlor oder Brom steht,

mit Azolen der Formel

Az - H (VII)

in welcher

Az die oben angegeben Bedeutung hat,

in Gegenwart eines organischen Lösungsmittels, beispielsweise Tetrahydrofuran, und in Gegenwart eines Säurebinders, beispielsweise Trimethylamin oder überschüssiges Azol, bei Temperaturen zwischen 0 und 80°C, vorzugsweise 0 und 40°C, umsetzt. Die Isolierung der Verbindungen der Formel (II) erfolgt dadurch, daß man das Reaktionsgemisch mit Wasser versetzt, den entstehenden Niederschlag abfiltriert, trocknet und gegebenenfalls durch Umkristallisation reinigt.

Die als Ausgangsstoffe verwendeten Hydroxamsäurehalogenide der Formel (VI) sind bekannt (vgl.H.Ulrich 'The Chemistry of Imidoyl Halides', Seiten 157-172, Plenum Press, New York 1968 und die dort zitierten Literaturstellen). Noch nicht bekannte lassen sich nach den dort beschriebenen Verfahren leicht herstellen, so z.B. durch Chlorierung der entsprechenden Aldoxime.

Die Oxime der Formel (II), in denen n für 1 steht, können hergestellt werden, indem man Azolylketone der Formel

$$R - \overset{O}{\overset{\|}{C}} - CH_2 - Az \qquad \text{(VIII)}$$

in welcher

Az und R die oben angegeben Bedeutung haben,

mit Hydroxylamin in Gegenwart eines Lösungsmittels, vorzugsweise Alkohole bzw. wäßrige Alkohole, bei Temperaturen zwischen 20 und 100°C, vorzugsweise zwischen 50 und 80°C, umsetzt. Dabei wird das Hydroxylamin vorzugsweise in Form seiner Salze, insbesondere als Hydrochlorid, in Gegenwart eines Säurebindemittels, wie beispielsweise Natriumcarbonat, eingesetzt. Die Isolierung der Verbindungen der Formel (II) erfolgt dadurch, daß man das während der Umsetzung entstehende Produkt nach Abdestillieren des Lösungsmittels nach üblichen Methoden aufarbeitet.

Die Azolylketone der Formel (VIII) können erhalten werden, indem man Halogenketone der Formel

$$R - \overset{O}{\overset{\|}{C}} - CH_2 - Y \qquad \text{(IX)}$$

in welcher

R und Y die oben angegebene Bedeutung haben,

mit Azolen der Formel (VII) in Gegenwart eines Verdünnungsmittels, beispielsweise Kaliumcarbonat, bei Temperaturen zwischen 20 bis 150°C, vorzugsweise 60 bis 120°C, umsetzt. Die Isolierung der Verbindungen der Formel (IX) erfolgt dadurch, daß man das während der Reaktion entstehende Salz abfiltriert und das Filtrat durch Abdestillieren des Lösungsmittels einengt. Der dabei zurückbleibende Feststoff wird getrocknet und durch Umkristallisation gereinigt.

Als Ausgangsstoffe der Formel (II) seien beispielsweise genannt:

3,3-Dimethyl-2-oximino-pyrazol-1-yl-butan
3,3-Dimethyl-1-imidazol-1-yl-2-oximino-butan
3,3-Dimethyl-2-oximino-1-(1,2,4-triazol-1-yl)-butan
3,3-Dimethyl-2-oximino-1-(1,2,3-triazol-1-yl)-butan
3,3-Dimethyl-2-oximino-1-(1,3,4-triazol-1-yl)-butan
1-Benzimidazol-1-yl-3,3-dimethyl-2-oximino-butan
1-Benztriazol-1-yl-3,3-dimethyl-2-oximino-butan
2-Oximino-pyrazol-1-yl-propan
2-Oximino-1-(1,2,4-triazol-1-yl)-propan
1-Imidazol-1-yl-2-oximino-propan
3-Methyl-2-oximino-1-pyrazol-1-yl-butan
1-Imidazol-1-yl-3-methyl-2-oximino-butan
3-Methyl-2-oximino-1-(1,2,4-triazol-1-yl)-butan
1-(4-Chlorpyrazol-1-yl)-3,3-dimethyl-2-oximino-butan
1-(2-Bromimidazol-1-yl)-3,3-dimethyl-2-oximino-butan
3,3-Dimethyl-1-(2-methylimidazol-1-yl)-2-oximino-butan
3,3-Dimethyl-2-oximino-(4-trifluormethylimidazol-1-yl)-butan
3,3-Dimethyl-1-(4-nitroimidazol-1-yl)-2-oximino-butan
1-(3-Chlor-1,2,4-triazol-1-yl)-3,3-dimethyl-2-oximino-butan
3,3-Dimethyl-1-(4-nitro-2-methyl-imidazol-1-yl)-2-oximino-butan
3,3-Dimethyl-1-(5-methyl-4-nitro-imidazol-1-yl)-2-oximino-butan
3,3-Dimethyl-2-oximino-1-(3-trichlormethyl-1,2,4-triazol-1-yl)-butan
1-(3-Brom-1,2,4-triazol-1-yl)-3,3-dimethyl-2-oximino-butan
3,3-Dimethyl-2-oximino-1-(2,4,5-tribrom-imidazol-1-yl)-butan
2,2-Dimethyl-1-oximino-pyrazol-1-yl-propan
2,2-Dimethyl-1-imidazol-1-yl-oximino-propan
2,2-Dimethyl-1-oximino-1-(1,2,4-triazol-1-yl)-propan
2,2-Dimethyl-1-oximino-1-(1,2,3-triazol-1-yl)-propan
2,2-Dimethyl-1-oximino-1-(1,3,4-triazol-1-yl)-propan

1-Benzimidazol-1-yl-2,2-dimethyl-1-oximino-propan
1-Benztriazol-1-yl-2,2-dimethyl-1-oximino-propan
1-Oximino-1-pyrazol-1-yl-äthan
1-Oximino-1-(1,2,4-triazol-1-yl)-äthan
1-Imidazol-1-yl-1-oximino-äthan
2-Methyl-1-oximino-1-pyrazol-1-yl-propan
1-Imidazol-1-yl-2-methyl-1-oximino-propan
2-Methyl-1-oximino-1-(1,2,4-triazol-1-yl)-propan
1-(4-Chlorpyrazol-1-yl)-2,2-dimethyl-1-oximino-propan
1-(2-Bromimidazol-1-yl)-2,2-dimethyl-1-oximino-propan
2,2-Dimethyl-1-(2-methylimidazol-1-yl)-1-oximino-propan
2,2-Dimethyl-1-oximino-(4-trifluormethylimidazol-1-yl)-propan
2,2-Dimethyl-1-(4-nitroimidazol-1-yl)-1-oximino-propan
1-(3-Chlor-1,2,4-triazol-1-yl)-2,2-dimethyl-1-oximino-propan
1-(3-methylthio-1,2,4-triazol-1-yl)-3,3-dimethyl-1-oximino-propan
2,2-Dimethyl-1-(5-methyl-4-nitro-imidazol-1-yl)-1-oximino-propan
2,2-Dimethyl-1-oximino-1-(3-trichlormethyl-1,2,4-triazol-1-yl)-propan
1-(3-Brom-1,2,4-triazol-1-yl)-2,2-dimethyl-1-oximino-propan
2,2-Dimethyl-1-oximino-1-(2,4,5-tribrom-imidazol-1-yl)-propan

Die außerdem für die erfindungsgemäße Umsetzung (a) als Ausgangsstoffe benötigten Carbamoylhalogenide sind durch die Formel (III) allgemein definiert. In dieser Formel stehen $\underline{R^1}$ und $\underline{R^2}$ vorzugsweise für die Reste, die bei den Verbindungen der Formel (I) bereits vorzugsweise genannt wurden.

Die sulfenylierten Carbamoylhalogenide der Formel (III) sind bekannt (vergleiche DE-OS 2 654 282) oder lassen sich nach allgemein üblichen und bekannten Verfahren herstellen. Man erhält sie z.B. durch Umsetzung der entsprechenden Carbamidsäurehalogenide der Formel

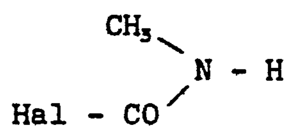

(X)

in welcher

Hal die oben angegebene Bedeutung hat,

mit den entsprechenden Sulfenchloriden der Formel

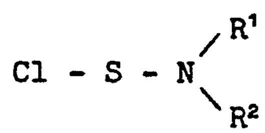

(XI)

in welcher

$R^1$ und $R^2$ die oben angegeben Bedeutung haben,

in Gegenwart eines säurebindenden Mittels ( vergleiche hierzu auch die Angaben in DE-AS 1 297 095, DE-OS 2 357 930, 2 409 463 und 2 654 282 sowie US-Patentschrift 3 939 192).

Als Ausgangsstoffe der Formel (III) seien beispielsweise genannt:

N,N'-Bis-(fluorcarbonyl)-thio-bis-methylamin

N-Methyl-N-morpholinylsulfenyl-carbamoylfluorid

1-Methylthioacetaldehyd-O-[N-methyl-N-(N'-methyl-N'-fluorformyl-aminosulfenyl)-carbamoyl]-oxim

N-Methyl-N-(N'-methyl-N'-methoxycarbonyl-aminosulfenyl)-carbamoylfluorid

N-Methyl-N-(N'-methyl-N'-äthoxycarbonyl-aminosulfenyl)-carbamoylfluorid

N-Methyl-N-[N'-methyl-N'-(2-isopropoxy-phenoxycarbonyl)-aminosulfenyl]-carbamoylfluorid

N-Methyl-N-[N'-methyl-N'-(3,5-dimethyl-4-dimethylamino-phenoxycarbonyl)-aminosulfenyl]-carbamoylfluorid

N-Methyl-N-[N'-methyl-N'-3,5-dimethyl-4-methylmercapto-phenoxycarbonyl)-aminosulfenyl]-carbamoylfluorid

N-Methyl-N-[N'-methyl-N'-(2-sek.-butyl-phenoxycarbonyl)-aminosulfenyl]-carbamoylfluorid

N-Methyl-N-[N'-methyl-N'-(3-methyl-4-dimethylamino-phenoxycarbonyl)-aminosulfenyl]-carbamoylfluorid

Die für die erfindungsgemäße Umsetzung (b) als Ausgangsstoffe zu verwendenden Verbindungen sind durch die Formel (V) allgemein definiert. In dieser Formel steht $\underline{R^8}$ vorzugsweise für die Gruppierungen $-O-R^3$ und $-NR^4R^5$, wobei $\underline{R^3}$, $\underline{R^4}$ und $\underline{R^5}$ vorzugsweise für die Reste stehen, die bei den Verbindungen der Formel (I) bereits vorzugsweise genannt wurden.

Die Verbindungen der Formel (V) sind allgemein bekannte Verbindungen der organischen Chemie. Als Beispiele seien genannt:
Methanol, Aethanol, Isopropanol, Allylalkohol, Propargylalkohol, Phenol; 2,2-Dimethyl-benzofuran-7-ol; 2,2-Dimethyl-1,3-benzodioxo-4-ol, Naphthol; 3,5-Dimethyl-4-dimethylamino-phenol; 3,5-Dimethyl-4-methylmercaptophenol; 2-sek.-Butyl-phenol; 3-Methyl-4-dimethylaminophenol; 2-Isopropoxy-phenol; 2-Chlor-4,5-dimethyl-phenol; 3-Isopropyl-5-methyl-phenol; 3,5-Di-tert.-butyl-phenol; 3,5-Dimethyl-4-bis-allyl-amino-phenol; 3,4-Dimethylphenol; 3-Methylphenol; 3,4,5-Trimethylphenol; 2-Chlorphenol; 2-Isopropylphenol; 3-tert.-Butylphenol; 2-Aethylthiomethylphenol.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV.Haupt- und der I. und II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.
Die Metallsalzkomplexe der Verbindungen der Formel (I) können in einfacher Weise nach üblichen Verfahren erhalten werden, so z.B. durch Lösen des Metallsalzes in Alkohol, z.B. Aethanol, und Hinzufügen zur Verbindung der Formel (I). Man kann Metallsalzkomplexe in bekannter Weise, z.B. durch Abfiltrieren, isolieren und gegebenenfalls durch Umkristallisation reinigen.

Als Verdünnungsmittel kommen für das erfindungsgemäße Verfahren (a) vorzugsweise alle inerten organischen Lösungsmittel infrage. Hierzu gehören vorzugsweise Ketone, wie Diäthylketon, insbesondere Aceton und Methyläthylketon; Nitrile, wie Propionitril, insbesondere Acetonitril; Alkohole, wie Aethanol oder Isopropanol;Aether, wie Tetrahydrofuran oder Dioxan; Formamide, wie insbesondere Dimethylformamid und halogenierte Kohlenwasserstoffe, wie Methylenchlorid, Tertachlorkohlenstoff oder Chloroform.

Die Umsetzungen gemäß Verfahren (a)und(b) werden in Gegenwart eines Säurebinders vorgenommen. Man kann alle üblicherweise verwendbaren anorganischen und organischen Säurebinder zugeben. Hierzu gehören vorzugsweise Alkalicarbonate, wie beispielsweise Natriumcarbonat, Kaliumcarbonat und Natriumhydrogencarbonat, ferner niedere tertiäre Alkylamine, Cycloalkylamine oder Arylalkylamine, wie beispielsweise Triäthylamin, N,N-Dimethyl-benzylamin, Dicyclohexylamin, weiterhin Pyridin und Diazabicyclooctan.

Die Reaktionstemperaturen können bei der Durchführung des Verfahrens (a) in einem größeren Bereich variiert werden. Im allgemeinen arbeitet man zwischen 0 und 100°C, vorzugsweise zwischen 10 und 80°C.

Bei der Durchführung des erfindungsgemäßen Verfahrens (a) arbeitet man vorzugsweise in molaren Mengen. Die Isolierung der Verbindungen der Formel (I) erfolgt in allgemein üblicher und bekannter Weise.

Für das erfindungsgemäße Verfahren (b) kommen als Verdünnungsmittel vorzugsweise inerte organische Lösungsmittel infrage. Hierzu gehören vorzugsweise Aether, wie Diäthyläther und Dioxan; aromatische Kohlenwasserstoffe, wie Benzol und Toluol; in einzelnen Fällen auch chlorierte Kohlenwasserstoffe, wie Chloroform, Methylenchlorid oder Tetrachlorkohlenstoff; ferner Ester, wie Essigsäureäthylester, sowie Hexamethylphosphorsäuretriamid. In manchen

Fällen kann auch ein Ueberschuß der Verbindung der Formel (V) verwendet werden.

Die Reaktionstemperaturen können bei der Durchführung des Verfahrens (b) in einem größeren Bereich variiert werden. Im allgemeinen arbeitet man zwischen 0 und 100°C, vorzugsweise zwischen 10 und 60°C.

Bei der Durchführung des Verfahrens (b) werden die Ausgangsstoffe vorzugsweise in molaren Mengen eingesetzt. Die Isolierung der Verbindungen der Formel (I) erfolgt nach üblichen Methoden.

Zum Teil ist es auch möglich, die einzelnen Stufen der Vorprodukte zur Herstellung der Oxime der Formel (II) oder der sulfenylierten Carbamoylhalogenide der Formel (III) sowie deren Umsetzungen zu den erfindungsgemäßen Stoffen ohne Isolierung der jeweiligen Zwischenprodukte in einer sogenannten Eintopfreaktion durchzuführen.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hyginesektor vorkommen. Sie sind gegen normal sensible und resistente Arten sowie gegen alle oder einezlne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A

Laphygma-Test

Lösungsmittel: 3 Gewichtsteile
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei bedeutet 100 %,daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 5, 6, 3, 4 und 2.

**Beispiel B**

Tetranychus-Test (resistent)

Lösungsmittel: 3 Gewichtsteile
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 6, 5, 3, 4 und 2.

Beispiel C

Grenzkonzentrations-Test / Wurzelsystemische Wirkung

Testinsekt: Phaedon cochleariae - Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracèa). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 3, 4, 6, 1 und 5

## Beispiel D

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 2, 3, 4, 6, 1 und [illegible].

**Beispiel E**

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 2, 3, 4, 6 und 1.

Herstellungsbeispiele

Beispiel 1

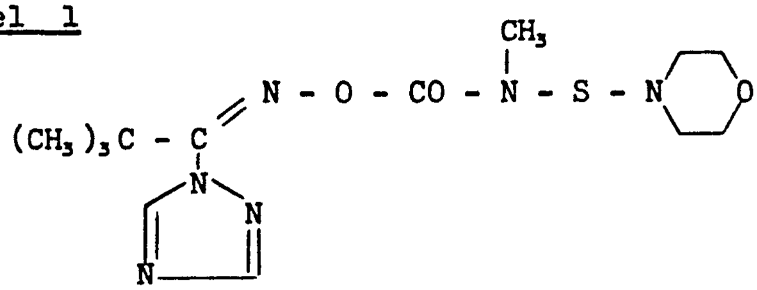

(Verfahren a)

8,4g (0,05 Mol) 2,2-Dimethyl-1-oximino-1-(1,2,4-triazol-1-yl)-propan und 9,7g (0,05 Mol) N-Methyl-N-morpholin-4-yl-sulfenyl-carbamoylfluorid werden in 100 ml absolutem Essigester vereinigt und unter Rühren mit 5,1g (0,05 Mol) Triäthylamin versetzt. Man läßt über Nacht stehen, wäscht die Essigesterphase mit Wasser, trocknet sie über Natriumsulfat und engt im Vakuum ein. Das zurückbleibende Oel wird mit Aether/Petroläther (1:1) zur Kristallisation gebracht. Man erhält 3,5g (20 % der Theorie) 2,2-Dimethyl-1-[(N-methyl-N-morpholin-4-yl-sulfenyl)-carbamoyloximino]-1-(1,2,4-triazol-1-yl)-propan vom Schmelzpunkt 93-94°C.

Herstellung der Ausgangsprodukte

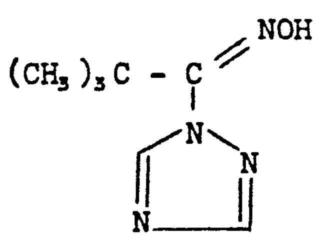

Zu einer Mischung von 69g (1 Mol) 1,2,4-Triazol und 101g (1 Mol) Triäthylamin in 500 ml absolutem Tetrahydrofuran wird bei 0 bis 3°C tropfenweise eine Lösung von 135,5 g (1 Mol) Pivalinhydroxamsäurechlorid in 100 ml absolutem Tetrahydrofuran gegeben. Nach Beendigung des Zutropfens wird 24 Stunden bei ca.20°C nachgerührt.
Anschließend versetzt man das Reaktionsgemisch mit 1,5 l

Wasser, saugt den Niederschlag ab, und kristallisiert aus Cyclohexan/Essigester um. Man erhält 106,5g (63 % der Theorie) 2,2-Dimethyl-1-oximino-1-(1,2,4-triazol-1-yl)-propan vom Schmelzpunkt 134 - 37°C.

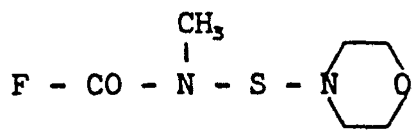

Zu einer Lösung von 77g (0,5 Mol) Morpholino-N-sulfenchlorid und 38,5g (0,5 Mol) N-Methylcarbamidsäurefluorid in 300 ml Toluol werden bei -10°C unter Rühren langsam 50,5 g (0,5 Mol) Triäthylamin eingetropft. Nach 2-stündigem Rühren bei 20°C wird die Toluolphase mehrmals mit Wasser gewaschen, über Natriumsulfat getrocknet und eingeengt. Der Rückstand kristallisiert nach dem Verreiben mit Petroläther . Man erhält 68g (70 % der Theorie) N-Methyl-N-morpholin-4-yl-sulfenyl-carbamoylfluorid vom Schmelzpunkt 48-50°C.

Beispiel 2

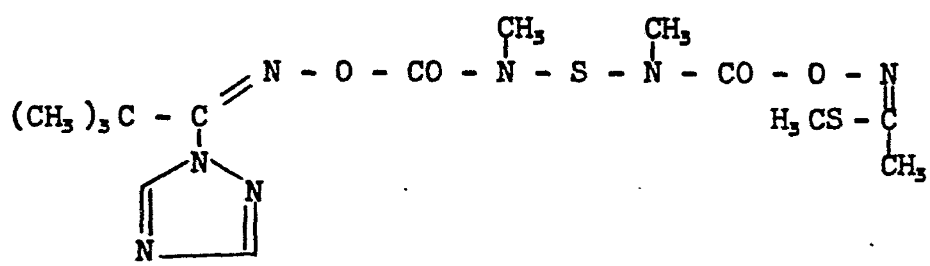

(Verfahren a)

2,87g (0,017 Mol) 2,2-Dimethyl-1-oximino-1-(1,2,4-triazol-1-yl)-propan und 4,6g (0,017 Mol) 1-Methylthio-acetaldehyd-O-[N-methyl-N-(N'-methyl-N'-fluorformyl-aminosulfenyl)-carbamoyl]-oxim werden in 50 ml absolutem Dioxan vereinigt und tropfenweise mit 2g (0,02 Mol) Triäthylamin versetzt. Nach 48-stündigem Stehen bei Raumtemperatur gießt man die Reaktionslösung auf 150 ml Wasser.

Die organische Phase wird mit Essigester extrahiert, über Natriumsulfat getrocknet und im Vakuum eingeengt. Der Rückstand wird aus Aether/Petroläther (1:1) umkristallisiert. Man erhält 3 g (42 % der Theorie) 2,2-Dimethyl-1-{N-methyl-N-[1-Methylthio-acetaldehyd-O-(methylcarbamoylsulfenyl)-oxim]-carbamoyloximino}-1-(1,2,4-triazol-1-yl)-propan vom Schmelzpunkt 95-98°C.

Herstellung des Ausgangsproduktes

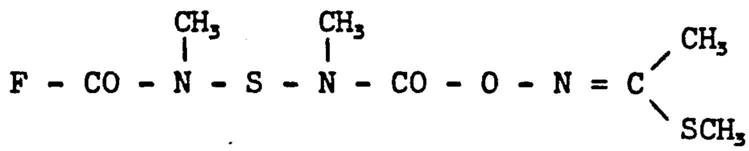

Durch Umsetzung von 1-Methylthioacetaldoxim mit Bis-(N-methyl-N-fluorcarbonylamino)-sulfid gemäß DE-OS 2 654 282; Schmelzpunkt 102-104°C.

Beispiel 3

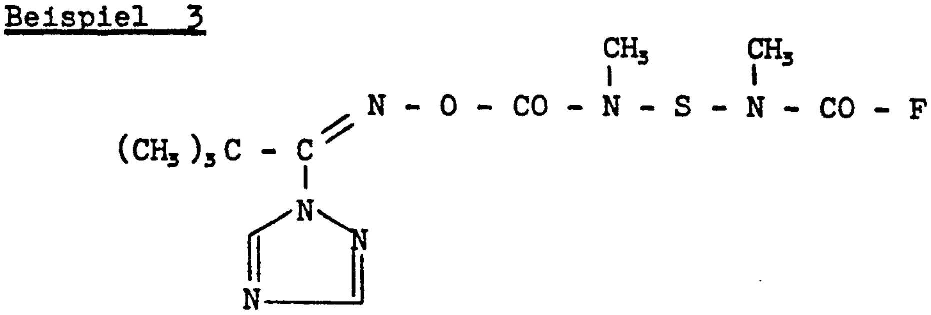

(Verfahren a)

16,8g (0,1 Mol) 2,2-Dimethyl-1-oximino-1-(1,2,4-triazol-1-yl)-propan und 18,4g (0,1 Mol) N,N'-Bis-(fluorcarbonyl)-thiobis-methylamin werden in 100 ml absolutem Dioxan gelöst und bei Raumtemperatur tropfenweise mit 10,1 g(0,1 Mol) Triäthylamin versetzt. Nach 2-stündigem Rühren wird die Reaktionsmischung auf 250 ml Wasser gegossen und mit Methylenchlorid extrahiert. Die organische Phase wird über Natriumsulfat getrocknet und im Vakuum eingeengt. Das zu-

rückbleibende Oel wird mit Aether/Petroläther (1:1) teilweise zur Kristallisation gebracht. Man erhält 4 g (12 % der Theorie) 2,2-Dimethyl-1-[N-methyl-N-(N'-methyl-N'-fluorcarbonyl-aminosulfenyl)-carbamoyloximino]-1-(1,2,4-triazol-1-yl)-propan vom Schmelzpunkt 82-85°C..

Beispiel 4

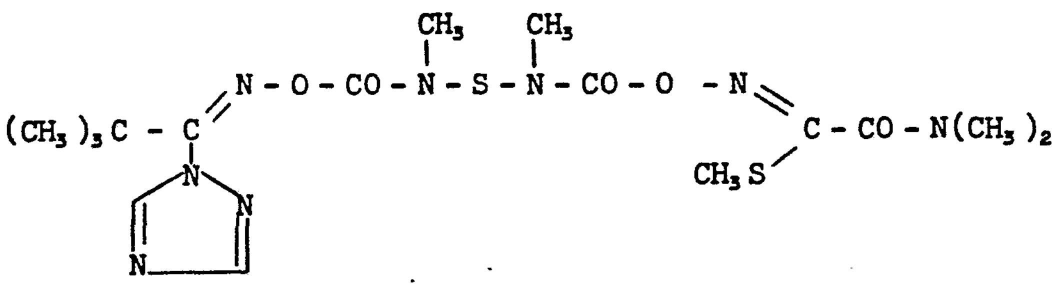

(Verfahren b)

9,9g (0,03 Mol) 2,2-Dimethyl-1-[N-methyl-N-(N'-methyl-N'-fluorcarbonyl-amino-sulfenyl)-carbamoyloximino]-1-(1,2,4-triazol-1-yl)-propan (Beispiel 3) und 4,9 g (0,05 Mol) α-Methylmercapto-α-oximino-essigsäure-dimethylamid werden in 50ml absolutem Dioxan bei 20°C unter Rühren mit 3g (0,03 Mol) Triäthylamin versetzt. Man läßt 24 Stunden bei Raumtemperatur rühren und gibt das Reaktionsgemisch auf Wasser. Danach wird mit Essigester extrahiert, die Essigester-Phase über Natriumsulfat getrocknet und eingeengt. Der Rückstand wird durch Zusatz von Aether zur Kristallisation gebracht. Man erhält 4,2g (30 % der Theorie) 2,2-Dimethyl-1-{N-methyl-N-[1-methylthio-1-dimethylcarbamoyl-acetaldehyd-O-(methylcarbamoyl-sulfenyl)-oxim]-carbamoyl-oximino}-1-(1,2,4-triazol-1-yl)-propan vom Schmelzpunkt 105-108°C.

In entsprechender Weise werden die Verbindungen der folgenden Tabelle 1 erhalten:

Tabelle 1

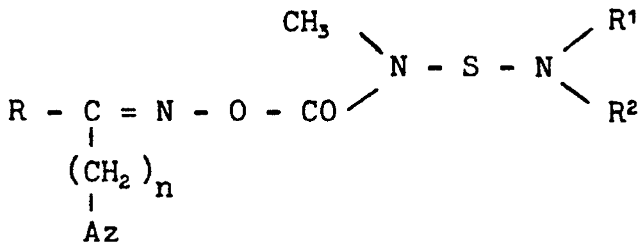

| Bsp. Nr. | Az | n | R | $R^1$ | $R^2$ | Schmelzpunkt (°C) |
|---|---|---|---|---|---|---|
| 5 | 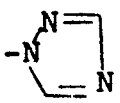 | 0 | $C(CH_3)_3$ | $CH_3$ | $-CO-O-C_2H_5$ | Oel |
| 6 | 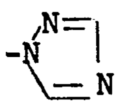 | 0 | $C(CH_3)_3$ | $CH_3$ | $-CO-O-CH_3$ | 102-03 |

Patentansprüche:

1. N,N'-unsymetrisch substituierte Thio-bis-amine der allgemeinen Formel I

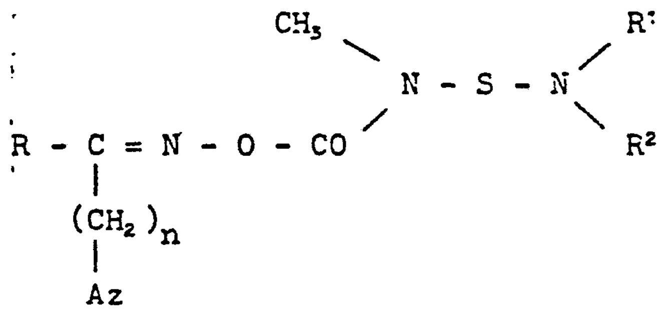
(I)

in welcher

Az für einen gegebenenfalls substituierten Azolylrest steht,

R für Alkyl steht,

$R^1$ für Alkyl steht,

$R^2$ für Alkyl, Halogencarbonyl oder die Gruppierungen $-CO-O-R^3$ und $-CO-NR^4R^5$ steht

$R^1$ und $R^2$ gemeinsam mit dem angrenzenden N-Atom für einen Ring stehen, der gegebenenfalls ein weiteres Heteroatom enthält, wobei $R^1$ für Methyl stehen muß, wenn $R^2$ für Halogencarbonyl steht,

$R^3$ für Alkyl, Alkenyl, Alkinyl, gegebenenfalls substituiertes Aryl oder die Gruppierung $-N=CR^6R^7$ steht,

$R^4$ und $R^5$ gleich oder verschieden sind und für Alkyl stehen oder gemeinsam mit dem angrenzenden N-Atom für einen Ring stehen, der gegebenenfalls ein weiteres Heteroatom enthält,

$R^6$ und $R^7$ gleich oder verschieden sind und für Wasserstoff, Alkyl, Alkoxy, Alkylthio, Alkoxyalkyl, Alkylthioalkyl, Dialkylaminocarbonyl und Alkoxycarbonyl oder gemeinsam mit dem angrenzenden C-Atom für einen Dithian-Ring stehen, und

n für O oder 1 steht, sowie deren physiologisch verträgliche Säureadditions-Salze und Metallsalz-Komplexe.

2. Verfahren zur Herstellung der N,N'-unsymmetrisch substituierten Thio-bis-amine der Formel (I), dadurch gekennzeichnet, daß man

a) Oxime der Formel II

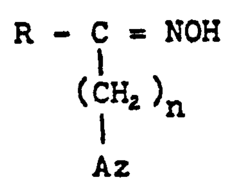

(II)

in welcher

Az, R und n die oben angegebene Bedeutung haben,

mit sulfenylierten Carbamoylhalogeniden der Formel III

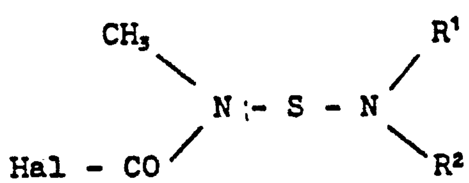

(III)

in welcher

$R^1$ und $R^2$ die oben angegebene Bedeutung haben, wobei aber $R^1$ nur für Methyl steht, wenn $R^2$ für Halogencarbonyl steht, und

Hal für Fluor oder Chlor steht,

in Gegenwart eines Verdünnungsmittels und eines Säurebindemittels umsetzt, oder

b) gegebenenfalls die nach dem Verfahren (a) erhältlichen sulfenylierten Oximcarbamate der Formel

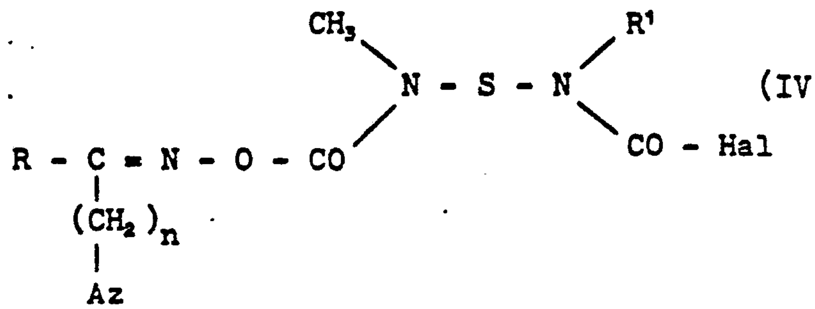

(IV)

in welcher

Az, R, $R^1$,
Hal und n die oben angegebene Bedeutung haben,

mit Verbindungen der Formel

$H - R^8$ (V)

in welcher

$R^8$ für die Gruppierungen $-O-R^3$ und $-NR^4R^5$ steht und $R^3$, $R^4$ und $R^5$ die oben angegebene Bedeutung haben,

in Gegenwart eines Verdünnungsmittels und eines Säurebindemittels umsetzt.

3. Insektizide, akarizide und nematizide Mittel, gekennzeichnet durch einen Gehalt an mindestens einem N,N'-unsymmetrisch substituierten Thio-bis-amin der Formel (I).

4. Verwendung von N,N'-unsymmetrisch substituierten Thio-bis-aminen der Formel (I) zur Bekämpfung von Insekten, Spinnentieren oder Nematoden.

5. Verfahren zur Bekämpfung von Insekten, Spinnentieren oder Nematoden, dadurch gekennzeichnet, daß man N,N'-unsymmetrisch substituierte Thio-bis-amine der Formel (I) auf Insekten, Spinnentieren oder Nematoden und/oder ihren Lebensraum einwirken läßt.

6. Verfahren zur Herstellung insektizider, akarizider oder nematizider Mittel, dadurch gekennzeichnet, daß man N,N'-unsymmetrisch substituierte Thio-bis-amine der Formel (I) mit Streckmitteln und/oder oberflächenaktiven Mitteln vermischt.

0005780

Europäisches Patentamt

# EUROPÄISCHER RECHERCHENBERICHT

Nummer der Anmeldung

EP 79 10 1538

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | DE - A - 2 613 167 (BAYER)<br>* Ansprüche *<br>-- | 1,3,4-6 | C 07 D 249/08<br>A 01 N 9/22 |
| D | DE - A - 2 635 883 (BAYER)<br>* Ansprüche *<br>---- | 1,3,4-6 | |

RECHERCHIERTE SACHGEBIETE (Int. Cl.²)

C 07 D 249/08
A 01 N 9/22

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 07-08-1979 | CREMERS |